# DEMANDE DE BREVET EUROPEEN

(11) **EP 0 544 559 A1**
(43) Date de publication de la demande: **02.06.1993**
(21) Numéro de dépôt: 92403076.0
(22) Date de dépôt: 16.11.1992
(51) Int. Cl.: F21M 1/00

(54) **Lampe d'éclairage chirurgical avec moyen automatique de réglage de la concentration des rayons lumineux sur le champ opératoire**

(30) Priorité: 25.11.1991 FR 9114622
(71) Demandeur: S.D.M.C. SA (SOCIETE DISTRIBUTION MATERIEL CHIRURGICAL), F-31025 Toulouse (FR)
(72) Inventeur: Agut, Gérard, F-65300 Uglas (FR)
(74) Mandataire: Ravina, Bernard

(57) **Abrégé**

La lampe d'éclairage chirugical comprend au moins un réflecteur (4) et au moins une source lumineuse (3) déplaçable par rapport au réflecteur par le moyen de réglage automatique de la concentration des rayons lumineux sur le champ opératoire.

Le sens de déplacement de la source lumineuse dans le réflecteur (4) est fonction du résultat de la comparaison par le moyen (14) entre la valeur algébrique actuelle de la valeur de la mesure de l'intensité lumineuse de la lumière réfléchie par le champ opératoire à celle de la mesure effectuée précédement. Le moyen (10) de mesure de l'intensité compr̂end notamment un organe (11) sensible à la lumière logé dans la poignée de préhension (5) à partir du touché de laquelle s'effectue un cycle de réglage dans le cadre duquel plusieurs mesures de l'intensité lumineuse et déplacements de la source lumineuse (3) sont effectuées conjointement au déplacement de la source lumineuse (3) pour parvenir à la concentration optimale.

## Description

La présente invention a pour objet une lampe d'éclairage chirurgical avec moyens automatiques de réglage de la concentration des rayons lumineux sur le champ opératoire.

Les lampes d'éclairage chirurgical sont constituées par un corps de lampe se présentant sous la forme d'une enveloppe obturée par une paroi transparente, dans lequel sont montés au moins une source d'éclairage et au moins un réflecteur en relation optique avec la dite source pour réfléchir vers le champs opératoire les rayons lumineux émis par la dite source en direction de la surface interne du boîtier.

Généralement, les lampes d'éclairage produisent sur le champ opératoire une tache lumineuse de forme ronde et l'intensité lumineuse maximale d'éclairement du champ opératoire est obtenue avec une tache d'un diamètre de valeur déterminée pour chaque type d'appareil.

Les lampes d'éclairage chirurgical sont équipées de moyens de réglage de la concentration des rayons lumineux afin d'obtenir un éclairage optimal du champ opératoire lesquels sont actionnables manuellement.

Ce réglage de la concentration est obtenu par modification de la position relative du réflecteur et de la source lumineuse l'un par rapport à l'autre et les moyens permettant cette modification sont manoeuvrés à partir de l'action sur une poignée stérilisable montée par le chirurgien sur la lampe d'éclairage et dans l'axe de cette dernière.

En raison du fait que cette poignée se situe dans le champ stérile, elle ne peut être montée et manipulée que par le chirurgien lui-même ou par tout autre personne opérant dans le champ stérile.

Pour d'autres types de lampes, le réglage de la concentration est opéré à partir de l'action sur des boutons de manoeuvres externes et latéraux au boîtier de lampe. Ces boutons de manoeuvres étant situés en dehors du champ stérile leur manoeuvre ne peut être effectuée que par des auxiliaires.

Au cours d'une intervention chirurgicale, il est fréquent de devoir changer la position de la lampe pour, par exemple, éclairer le champ opératoire suivant un autre angle. Ce changement de position, dans la mesure où il implique une variation de distance entre le champ opératoire éclairé et la lampe, s'accompagne d'un agrandissement de la plage éclairée et d'un moindre éclairage du champ opératoire.

Le chirurgien ou bien ses auxiliaires devront procéder par voie manuelle à un nouveau réglage de la concentration de rayons lumineux.

Dans le premier cas de figure (réglage de la concentration par le chirurgien) le chirurgien est obligé d'interrompre sa tache pour procéder à ce réglage.

Dans le second cas de figure, il n'est pas possible d'obtenir un réglage précis, en raison du fait que les auxiliaires doivent demeurer en dehors de la zone stérile.

La présente invention a pour objet de résoudre les problèmes sus-évoqués en mettant en oeuvre une lampe d'éclairage chirurgical dotée de moyens de réglage automatique de la concentration des rayons lumineux sur le champ opératoire lesquels moyens sont activés à partir de l'action sur un élément monté sur la lampe et sensible au touché ou la pression de la main du chirurgien.

A cet effet, la lampe d'éclairage chirurgical selon la présente invention, comportant un corps (1) creux monté en fixation sur une structure porteuse, doté d'une ouverture obturée par une paroi (2) transparente, dans lequel sont montés au moins une source lumineuse (3) fixée à une structure support (3A) et au moins un réflecteur (4) en relation optique avec la source lumineuse pour réfléchir vers le champ opératoire les rayons lumineux, émis par la source lumineuse et éclairer le dit champ opératoire, lesquels rayons lumineux pour parvenir sur le dit champ traversent la paroi transparente (2), des organes de préhension manuelle(5) et (6) étant prévus sur la lampe de façon à pouvoir déplacer la lampe et ajuster sa position par rapport au champ opératoire et la source lumineuse (3) et le réflecteur (4) étant montés dans le corps de lampe (1) avec possibilité d'être déplacés l'un par rapport à l'autre par des moyens de réglage de la concentration des rayons lumineux sur le champ opératoire qui ajustent la position de la source lumineuse (3) et du réflecteur (4) l'un par rapport à l'autre de façon que l'intensité lumineuse de l'éclairage du dit champ soit optimale se caractérise essentiellement en ce que le moyen de réglage automatique de la concentration des rayons lumineux sur le champ opératoire est constitué par :
- un moyen (7) sensible au touché manuel de l'opérateur, comportant au moins un élément destiné à être touché, manuellement par l'opérateur, le dit moyen (7) délivrant lorsque le dit élément est touché un signal électrique d'activation de l'opération de réglage de la concentration des rayons lumineux,
- un moyen (9) de conduite de l'opération de réglage de la concentration des rayons lumineux recevant du moyen (7) le signal d'activation de l'opération de réglage de la concentration des rayons lumineux qui n'est conduite par le moyen (9) qu'après disparition du signal d'activation, cette disparition résultant du relachement de l'organe sensible au touché,
- un moyen (10) de mesure de l'intensité lumineuse de la lumière réfléchie sur le champ opératoire, activé par le moyen (9) de conduite et comportant un organe (11) sensible à la lumière, placé en regard et à distance du champ opératoire, délivrant une tension électrique dont la valeur est représentative de la valeur de l'intensité lumineuse de la lumière qu'il reçoit du champ opératoire,
- au moins un premier élément (12) de mémoire couplé au moyen (10) de mesure, activé par le moyen (9) de conduite de l'opération de réglage et dans lequel, lorsqu'il est activé, est enregistrée la valeur algébrique de la mesure effectuée par le dispositif de mesure,
- au moins un second élément (13) de mémoire activé par le moyen (9) de conduite de l'opération de réglage et dans lequel est enregistrée la valeur algébrique de la dernière mesure effectuée précédemment par le moyen (10) de mesure,
- un moyen comparateur (14) activé par le moyen (9) de conduite de l'opération de réglage connecté par ses entrées aux éléments (12), (13) de mémoire et par sa sortie au moyen (9) de conduite, le dit moyen comparateur (14) lorsqu'il est activé comparant les deux valeurs enregistrées dans les deux éléments de mémoire (12), (13) et délivrant au moyen (9) de conduite un signal représentatif de la valeur de la différence algébrique entre la valeur de la mesure enregistrée dans la première mémoire et la valeur de la mesure enregistrée dans la seconde mémoire,
- un moyen moteur (15) commandé par le moyen (9) de conduite de l'opération de réglage, couplé mécaniquement à la structure support (3A) de la source lumineuse (3) et/ou au réflecteur (4) pour les déplacer l'un par rapport à l'autre et dans un sens ou dans l'autre, le sens de déplacement étant déterminé par le moyen (9) de conduite,
- le dit moyen (9) de conduite, au cours d'une même opération de réglage de la concentration des rayons lumineux activant successivement:
   a)- le moyen (10) de mesure de l'intensité,
   b)- le premier élément (12) de mémoire,
   c)- le moyen comparateur(14),
   d)- le moyen moteur (15) et,
   e)- les premier et second éléments de mémoires (12), (13) pour enregistrer dans le second élément 13 de mémoire la valeur contenue dans le premier élément (12) de mémoire,
- et le dit moyen (9) de conduite tant que le signal délivré par le moyen de comparaison marque une différence positive ou nulle, répétant les opérations a, b, c, et e tout en maintenant activé le moyen moteur (15) de façon à déplacer de manière continue la source de lumière (3) par rapport au réflecteur (4), ce mouvement de déplacement étant interrompu, par désactivation du moyen moteur (15) dès que le signal délivré par le moyen de comparaison marque une différence négative.

L'opération de règlage pourra être interrompue dès que le moyen moteur (15) sera désactivé mais selon une caractéristique additionnelle de l'invention, l'opération de règlage est poursuivie et le moyen de conduite (9) par réactivation du moyen moteur (15) pendant une durée de temps prédéterminée commande le déplacement en sens inverse de la source lumineuse par rapport au réflecteur, et au terme de cette durée désactive le moyen moteur et interrompt l'opération de réglage de la concentrention.

Selon une autre caractéristique de l'invention la durée de réactivation du moyen moteur (15) est en relation avec le retard induit par les circuits électroniques de traitement des signaux.

Selon une autre caractéristique de l'invention en début de l'opération de règlage de la concentration, la source lumineuse est déplacée par rapport au réflecteur suivant un sens prédéterminé, et le moyen de conduite, par commande appropriée du moyen moteur (15) inversera ce sens de déplacement si le signal délivré par le moyen comparateur (14) marque une différence négative.

D'autres avantages et caractéristiques de l'invention apparaitront à la lecture de la description d'une forme préférée de réalisation donnée à titre d'exemple non limitatif en se référant aux dessins annexés en lesquels :
- la figure 1 est une vue schématique d'une lampe selon l'invention,
- la figure 2 est une vue de détail d'une lampe conforme à l'invention,

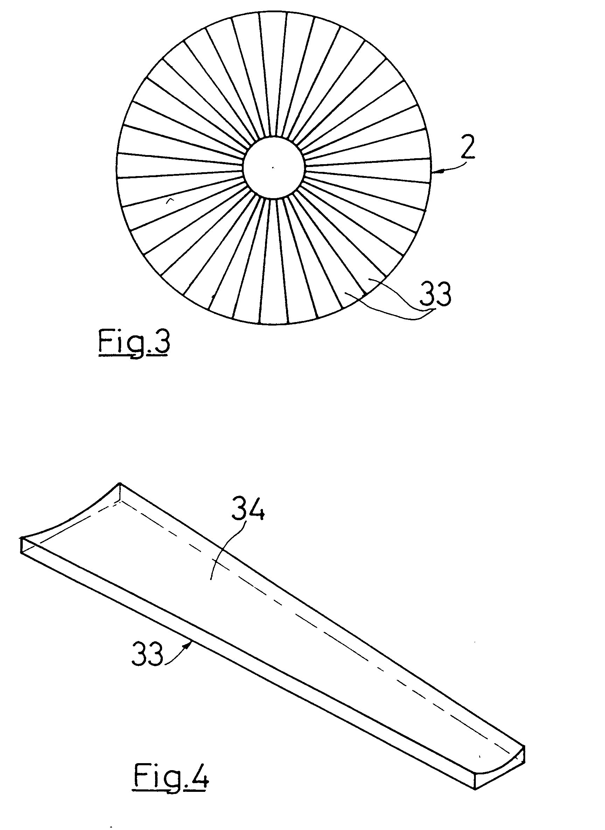

- la figure 3 est une vue en plan de la paroi transparente de la lampe,
- la figure 4 est une vue en perspective d'un élément de la paroi transparente de la lampe.

Telle que représentée la lampe d'éclairage chirugical selon l'invention comprend un corps de lampe creux 1 monté en fixation sur une structure porteuse doté d'une ouverture obturée par une paroi transparente 2, dans lequel sont montés au moins une source lumineuse 3 montée sur un support 3A et au moins un réflecteur 4 en relation optique avec la source lumineuse pour réfléchir vers le champ opératoire les rayons lumineux émis par la source lumineuse et éclairer le champ opératoire, lesdits rayons lumineux pour parvenir au champ opératoire traversant la paroi 2 transparente.

La lampe est équipée d'organes 5 et 6 de préhension manuelle de façon à pouvoir être déplacée par rapport au champ opératoire.

Par ces organes est ajustée également la position de la lampe par rapport au champ.

La source lumineuse 3 et/ou le réflecteur 4 sont montés dans le corps de lampe avec possibilité d'être déplacés l'un par rapport à l'autre par des moyens de réglage de la concentration des rayons lumineux sur le champ opératoire qui ajustent la position de la source lumineuse 3 et du réflecteur 4 l'un par rapport à l'autre de façon que le champ opératoire soit éclairé par une lumière d'intensité lumineuse maximale.

La paroi transparente 2 est pourvue d'un orifice central cylindrique par la périphérie duquel elle se fixe à une platine centrale 1A montée en fixation dans le corps de lampe. Par ailleurs, par sa bordure externe périmétrique, la paroi transparente se fixe à la bordure de l'ouverture du corps de lampe.

La lampe selon l'invention est également équipée de sources d'énergie électriques connectées aux différents équipements électriques qu'elle comporte de façon à assurer leur alimentation en énergie électrique.

Conformément à l'invention, le moyen de réglage automatique de la concentration des rayons lumineux sur le champ opératoire est constitué par :
- un moyen (7) sensible au touché manuel de l'opérateur, comportant au moins un élément destiné à être touché manuellement par l'opérateur, le dit moyen (7) délivrant lorsque le dit élément est touché un signal électrique d'activation de l'opération de réglage de la concentration des rayons lumineux,
- un moyen (9) de conduite de l'opération de réglage de la concentration des rayons lumineux recevant du moyen (7) le signal d'activation de l'opération de réglage de la concentration des rayons lumineux qui n'est conduite par le moyen (9) qu'après disparition du signal d'activation,cette disparition résultant du relachement de l'organe sensible au touché,
- un moyen 10 de mesure de l'intensité lumineuse de la lumière réfléchie sur le champ opératoire activé par le moyen 9 de conduite et comportant un organe 11 sensible à la lumière placé en regard et à distance du champ opératoire délivrant une tension électrique dont la valeur est représentative de la valeur de l'intensité lumineuse de la lumière qu'il reçoit du champ opératoire,
- un premier élément 12 de mémoire couplé au dispositif de mesure, activé par le moyen 9 de conduite de l'opération de réglage et dans, lequel lorsqu'il est activé, est enregistrée la valeur algébrique de la mesure effectuée par le dispositif de mesure,
- un second élément 13 de mémoire activé par le moyen 9 de conduite de l'opération de réglage et dans lequel lorsqu'il est activé, est enregistrée la valeur algébrique de la dernière mesure effectuée précédemment par le moyen 10 de mesure,
- un moyen comparateur 14 activé par le moyen 9 de conduite de l'opération de réglage connecté par ses entrées aux éléments 12, 13 de mémoire et par sa sortie au moyen 9 de conduite, le dit moyen comparateur (14) lorsqu'il est activé comparant les deux valeurs enregistrées dans les deux éléments de mémoire 12, 13 et délivrant au moyen 9 de conduite un signal représentatif de la valeur de la différence algébrique entre la valeur de la mesure enregistrée dans la première mémoire et la valeur de la mesure enregistrée dans la seconde mémoire,
- un moyen moteur 15 commandé par le moyen 9 de conduite de l'opération de réglage, couplé mécaniquement à la structure support 3A de la source lumineuse 3 et/ou au réflecteur 4 pour les déplacer l'un par'rapport à l'autre et dans un sens ou dans l'autre, le sens de déplacement étant déterminé par le moyen (9) de conduite,
- le dit moyen 9 de conduite au cours d'une même opération de réglage de la concentration des rayons lumineux activant sucessivement:
   a)- le moyen 10 de mesure de l'intensité,
   b)- le premier élément 12 de mémoire,
   c)- le moyen comparateur 14,
   d)- le moyen moteur 15 et,
   e)- les premier et second éléments de mémoires 12, 13 pour enregistrer dans le second élément 13 de mémoire la valeur contenue dans le premier élément 2) de mémoire,
- et le dit moyen (9) de conduite tant que le signal délivré par le moyen de comparaison marque une différence positive ou nulle, répétant les opérations a,b,c, et e tout en maintenant activé le moyen moteur (15) de façon à déplacer de manière continue la source de lumière (3) par rapport au réflecteur (4), ce mouvement de déplacement étant interrompu, par désactivation du moyen moteur (15) dès que le signal délivré par le moyen de comparaison marque une différence négative.

La délivrance par le moyen comparateur 14 d'un signal marquant une différence négative signifie que la position optimale de la source lumineuse par rapport au réflecteur, conduisant à une concentration maximale des rayons lumineux sur le champ opératoire est dépassée. Dans la mesure où la position obtenue est très proche de la position optimale il n'est pas génant d'interrompre à ce niveau l'opération de réglage de la concentration.

Cependant selon une-variante de réalisation l'opération de réglage, suite à l'apparition du signal marquant une différence négative se poursuit par déplacement en sens inverse de la source lumineuse par rapport au réflecteur et ce pendant une durée prédéterminée pour approcher ou atteindre la position optimale.

Cette durée pourra notamment tenir compte du retard induit par les différents organes électroniques des circuits de traitement des signaux.

En tout début de l'opération d réglage de la concentration, le moyen de conduite 9 commande le déplacement de la source lumineuse 3 par rapport au réflecteur 4 suivant un sens prédéterminé. Si ce sens de déplacement conduit à la délivrance par le moyen comparateur 14 d'un signal significatif d'une différence négative le moyen de conduite, par commande appropriée du moyen moteur 15 inversera le sens de déplacement de la source lumineuse 3 par rapport au réflecteur 4.
Ensuite l'opération de réglage de la concentration se poursuivra comme précédement décrit.

Selon la forme préférée de réalisation, l'élément destiné à être touché est constitué par un des organes 5, 6 de préhension manuelle.

Ainsi le réglage de la concentration des rayons lumineux sur le champ opératoire s'effectue dès que la lampe est en position.

Suivant une forme préférée de réalisation, l'organe de préhension manuelle constituant l'élément destiné à être touché est constitué par une poignée 5 cylindrique, stérilisable, fixée de manière amovible à la lampe et se développant suivant l'axe de cette dernière et sous la paroi transparente.

Cette poignée étant située dans le champ stérile, elle n'est destinée à être utilisée que par le chirurgien ou toute autre personne évoluant dans le champ stérile.

Selon une variante l'organe de préhension manuelle destiné à être touché pour déclencher l'opération de réglage automatique de la concentration des rayons lumineux est une anse 6 fixée au corps de lampe.

Cette anse située en dehors du champ stérile, sa préhension ne peut être effectuée que par un auxilaire ou toute autre personne évoluant en dehors du champ stérile.

On pourra également prévoir que le moyen 7 sensible au touché comporte deux éléments destinés à être touchés lesquels seront constitués par la poignée 5 et l'anse 6.

Ainsi le réglage de la concentration des rayons lumineux sur le champ opératoire pourra être effectuée aussi bien à partir de la saisie de la poignée par le chirurgien que de la saisie de l'anse par un de ses auxiliaires.

Préférentiellement l'élément destiné à être touché du moyen sensible au touché est une matière électriquement conductrice et est isolé de la masse de la lampe.

De plus il faut préciser que le réseau d'alimentation en énergie électrique des différents appareils de la salle d'opération crée un champ électrique dans lequel est situé l'élément destiné à être touché du moyen sensible au touché.

Cet élément sensible au touché est connecté électriquement à un circuit électronique du moyen 7 lequel circuit est apte à détecter toute variation de l'intensité du courant électrique qui le traverse résultant du touché de l'élément destiné à être touché du moyen 7 sensible au touché.

Selon la forme préférée de réalisation le circuit électronique du moyen 7 comprend un filtre connecté électriquement par son entrée à l'élément destiné à être touché du moyen 7 et par sa sortie à un redresseur de courant constitué par exemple par un convertisseur fréquence/tension. Le filtre est conçu de façon à ne laisser passer que les courants de frequence de valeur égale à cinquante ou soixante Hertz. A la sortie du convertisseur fréquence/tension est connectée une des entrées d'un comparateur à seuil dont l'autre entrée est connectée électriquement à une source de tension de référence.

Par sa sortie ce comparateur est connecté au moyen 9 de conduite de l'opération de réglage de la concentration. Le comparateur à seuil ne délivrera un signal que si la valeur de la tension délivrée par le convertisseur est au moins égale à la valeur de la tension de référence.

De cette façon est évité tout déclenchememnt intempestif de l'opération de réglage de la concentration, en l'absence de tout touché de l'élément sensible au touché 5 et/ou 6.

Le moyen de conduite 9 de l'opération de réglage de la concentration des rayons lumineux sur le champ opératoire est, par exemple, constitué par une unité centrale de traitement 16 connecté par l'intermédiaire de bus de données, de commandes et d'adresses à des unités d'entrées, de sorties, à des mémoires vives (RAM) 17 et mortes 18 (ROM) contenant notamment le programme de réglage automatique de la concentration des rayons lumineux.

L'unité centrale de traitement 16 pourra être constitué par un micro-processeur de tout type connu et adapté à la présente application.

L'unité centrale de traitement 16, par une des ses entrées est connectée au circuit électronique du moyen 7 sensible au touché pour recevoir par cette entrée le signal d'activation de l'opération de réglage de la concentration des rayons lumineux, laquelle ne se déroulera qu'à partir de l'instant où ce signal disparaitra.

Dès que ce signal disparait le moyen de conduite 9 délivre au moyen 10 de mesure un signal d'activation.

Selon la forme préférée de réalisation, l'organe 11 sensible à la lumière du moyen 10 de mesure de l'intensité lumineuse de la lumière réfléchie sur le champ opératoire, est monté en fixation sur une platine 19 et ce dans l'axe de la lampe sous la paroi transparente 2.

Cette disposition permet de dégager totalement l'espace entre la lampe et le champ opératoire.

Avantageusement, la poignée stérilisable est creuse, est pourvue d'une ouverture en regard du champ opératoire et vient lors de la fixation sur la lampe entourer l'organe 11 sensible à la lumière.

Préférentiellement, la platine 19 sera fixée par tout moyen connu de l'homme de l'art à la platine 1A.

A cette platine 1A se fixera de manière amovible, par tout moyen connu de l'homme de l'art, la poignée stérilisable 5.

Il faut noter que le creux formé dans la poignée stérilisable destiné à loger l'élément sensible 11 s'étend sur toute la longueur de ladite poignée et que cette dernière est pourvue d'organes de centrage coopérant en liaison de forme avec des organes de centrage montés sur la platine 1A.

Cette disposition assure la parfaite coaxialité de la poignée tant par rapport à la lampe que par rapport à l'élément 11 sensible à la lumière du moyen 10 de mesure.

A titre d'exemple purement indicatif, l'organe 11 sensible à la lumière sera constitué par une cellule photo-électrique et le moyen 10 de mesure de l'intensité lumineuse des rayons réfléchis par le champ opératoire comprendra notamment un convertisseur analogique/numérique de façon que la valeur de la mesure effectuée soit délivrée au premier élément 12 de mémoire sous forme de code binaire.

Ce convertisseur analogique/numérique sera connecté par son entrée à l'organe 11 sensible à la lumière et par ses sorties au bus de données du moyen 9 de conduite de l'opération de réglage automatique de la concentration.

Selon une forme préférée de réalisation entre l'organe 11 sensible à la lumière et le convertisseur analogique numérique sera disposé un circuit de traitement du signal constitué par exemple par un écreteur, suivi d'un moyenneur, suivi d'un intégrateur.

Avantageusement, le premier élément de mémoire 12 ainsi que le second élément 13 de mémoire sont respectivement deux éléments de la mémoire vive 17 associée à l'unité centrale de traitement 16.

Le moyen comparateur 14 sera de tout type connu.

En variante, la fonction de comparaison entre la valeur enregistrée dans le premier élément 12 de mémoire et la valeur enregistrée dans le second élément 13 de mémoire sera assurée par l'unité centrale de traitement 16.

Cette disposition est de nature à simplifier le moyen de réglage de la concentration des rayons lumineux.

Le moyen moteur 15 est comme dit précédemment commandé par le moyen 9 de conduite de l'opération de réglage et est couplé mécaniquement à là structure support 3A de la source lumineuse 3 et/ou au réflecteur 4 pour les déplacer l'un par rapport à l'autre.

De plus, la source lumineuse 3 et le réflecteur 4 sont déplacés l'un par rapport à l'autre dans un sens ou dans l'autre, le sens du déplacement étant déterminé par le moyen de conduite 9 en fonction du résultat de la comparaison effectué par le moyen comparateur 14.

Les moyens moteurs comprennent un moteur électrique 20 à courant continu par exemple dont l'arbre de sortie rotatif est connecté par l'intermédiaire d'une transmission mécanique de mouvement à la platine 3A de la source lumineuse et/ou au réflecteur.

Le moteur électrique dont l'arbre de sortie peut être mû dans un sens ou dans l'autre en fonction de la polarité de la tension électrique appliquée à ses bornes électriques d'entrée est connecté par lesdites bornes d'entrées à un circuit électrique 21 de puissance, activé et commandé par le moyen 9 de conduite de l'opération de réglage.

A titre d'exemple, purement indicatif, le circuit de puissance sera connecté à une source d'énergie électrique par l'intermédiaire d'un organe interrupteur commandé dans le sens de l'ouverture ou de la fermeture, par des impulsions électriques de commande délivrées par le moyen 9 de conduite de l'opération de réglage.

Par ailleurs, ce circuit de puissance comprendra un organe inverseur du sens de polarité de la tension électrique d'alimentation du moteur électrique. Cet organe inverseur sera également activé pour l'inversion de la polarité par des impulsions électriques délivrées par le moyen 9 de conduite de l'opération de réglage.

Le moyen de réglage de la concentration des rayons lumineux peut équiper tout type de lampe d'éclairage chirurgical dont la position de la source lumineuse 3 par rapport au réflecteur 4 est ajustable.

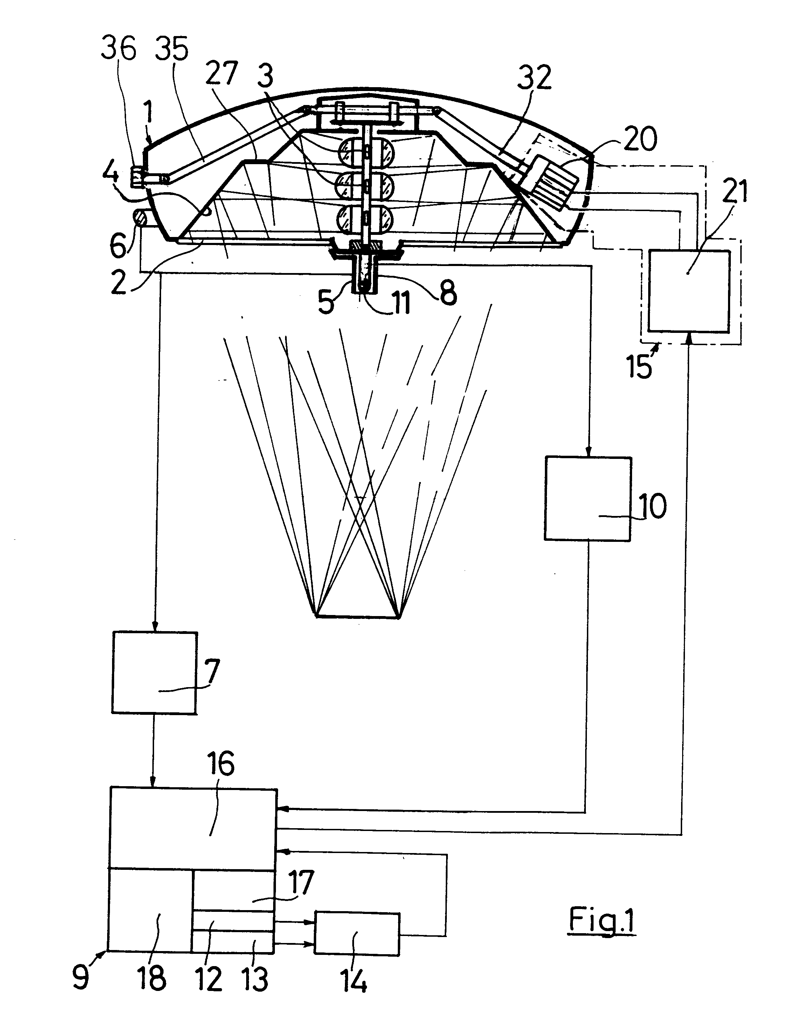

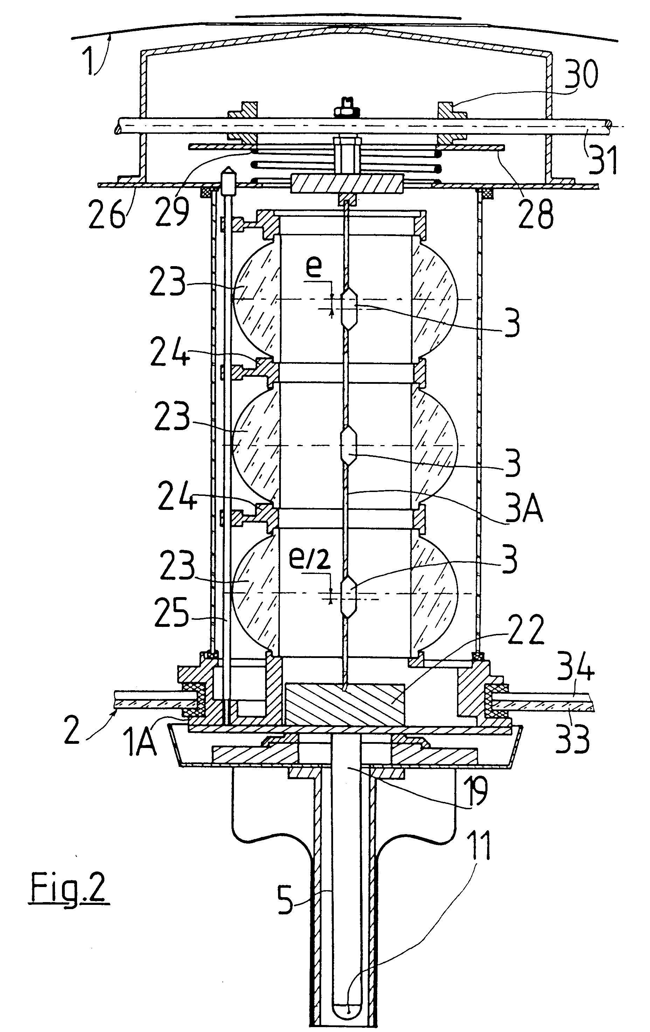

En figures 1 et 2 est représentée une telle lampe. La lampe selon cette figure comprend au moins une source lumineuse 3 déplaçable et au moins un réflecteur 4 monté de manière fixe dans le corps de lampe 1.

Préférentiellement, la lampe selon ces figures est équipée dans l'axe de symétrie du corps de lampe, par exemple de trois sources lumineuses 3 montées à écartement les unes des autres suivant l'axe de symétrie du corps de lampe sur une structure support commune 3A s'étendant dans le corps de lampe 1 suivant l'axe de symétrie de ce dernier.

La structure support 3A comprend au moins deux montants, parallèles à l'axe longitudinal de la lampe, espacés l'un de l'autre et maintenus à écartement l'un de l'autre par des entretoises.

Les sources lumineuses sont montées en fixation entre les montants de la structure support.

La zone terminale basse de la structure support comprend une embase à laquelle se fixent les montants. Cette embase s'engage dans un logement de guidage en translation pratiqué dans la platine centrale 1A. Par sa zone terminale haute, la structure support coopére avec des moyens d'actionnement en translation suivant l'axe de symétrie du corps de lampe.

Autour de chaque source lumineuse 3 est disposé un manchon optique 23 présentant intérieurement une surface cylindrique d'axe confondu avec l'axe de symétrie du corps de boîtier et extérieurement une surface de révolution convexe.

La lampe est donc équipée d'autant de manchons optiques coaxiaux qu'elle comporte de sources lumineuses. Les manchons sont fixés à des entretoises d'expacement 24 lesquelles sont rigidement fixées à une structure porteuse fixée dans le corps de boîtier.

Cette structure porteuse pourra être constituée par des tirants 25 fixés par leur zone terminale basse à la platine centrale 1A et par leur zone supérieure haute à une paroi supérieure 26 fixée dans le corps de lampe.

Par modification de la position axiale de la source lumineuse dans le manchon optique correspondant est modifiée par rapport à l'axe de symétrie du manchon, l'orientation du faisceau lumineux issu dudit manchon et par conséquent l'angle de réflexion des rayons lumineux sur le réflecteur 4.

Toujours selon la forme préférée de réalisation, la lampe telle que représentée en figure 1 est équipée par exemple de deux réflecteurs 4 à surface réfléchissante tronconiques, coaxiaux, montés dans le corps de lampe l'un au dessus de l'autre et autour des manchons optiques, ces dits réflecteurs et ces dits manchons étant coaxiaux.

Le réflecteur supérieur est destiné à réfléchir les rayons lumineux de la source lumineuse supérieure et de son manchon optique tandis que le réflecteur inférieur réfléchi les rayons lumineux des deux autres sources lumineuses et de leur manchon optique associé.

Le réflecteur supérieur est fixé par sa bordure supérieure à la paroi supérieure 26 et par sa bordure inférieure à une paroi médiane 27 en forme de courone à laquelle se fixe le réflecteur inférieur par sa bordure supérieure.

Par sa bordure inférieure le réflecteur inférieur se fixe à la bordure de l'ouverture du corps de lampe 1.

Préférentiellement, l'angle au sommet de la surface conique suivant laquelle se développe la surface réfléchissante du réflecteur supérieur est beaucoup plus important que l'angle au sommet de la surface conique suivant laquelle se développe la surface réfléchissante de l'autre réflecteur.

En outre, le diamètre de la bordure inférieure du réflecteur supérieur est plus faible que le diamètre de la bordure supérieure de l'autre réflecteur.

De plus, lorsque l'une des sources lumineuses 3 est placée dans le plan de symétrie de son manchon optique les deux autres sont décalées par rapport au plan de symétrie de leur manchon respectif. Ce plan de symétrie étant celui perpendiculaire à l'axe du corps de lampe.

Suivant la forme de réalisation objet de la figure 1 sur laquelle apparaissent trois sources lumineuxes, trois manchons optiques et deux réflecteurs, on remarquera que la source lumineuse médiane est placée suivant le plan de symétrie de son manchon tandis que la source lumineuse supérieure est en dessous du plan de symétrie de son manchon et écartée d'une valeur égale à e. La source lumineuse inférieure est en dessous du plan de symétrie de son manchon et est écartée du dit plan d'une valeur égale à e/2.

Grâce à ces caractéristiques les sources lumineuses 3 en association avec leur manchon optique et leur réflecteur produisent des faisceaux lumineux qui quel que soit la position des sources lumineuses dans leur manchon optique respectif viendront se superposer ou se mélanger suivant un plan géométrique unique perpendiculaire à l'axe de la lampe.

Il faut noter que projetés sur ce plan géométrique unique les faisceaux forment chacun une tache lumineuse de même diamètre que celle formée par les deux autres.
Ainsi on comprend qu'à ce plan de superposition correspond une concentration maximale de rayons lumineux.
La distance de ce plan par rapport à la lampe dépend de la position des sources lumineuses dans leur manchon.

Ainsi par déplacement axial des sources lumineuses 3 est obtenu le déplacement de ce plan de superposition.

Le réglage de la concentration consistera à faire coïncider la position de ce plan de superposition avec celle du champ opératoire.

Comme dit précédemment la structure support 3A coopère par sa zone terminale haute avec les moyens d'entrainement en translation suivant l'axe de symétrie du corps de lampe, ces dits moyens étant actionnés par l'intermédiaire de la transmission du mouvement par le moteur électrique 20.

Selon la forme préférée de réalisation, les moyens d'actionnement en translation comprennent, fixée, à la zone terminale de la structure 3A, une plaque de poussée 28 repoussée sous l'action d'un organe élastique 29 contre la bordure périmétrique d'au moins un excentrique 30 calé à rotation sur un arbre 31 monté à rotation dans deux paliers et accouplé par l'intermédiaire d'un joint cardan à une tringle d'actionnnement 32 accouplée par l'intermédiaire d'un joint cardan à l'arbre de sortie du moteur 20.

La tringle d'actionnement constitue la transmission sus-évoquée.

Le moteur électrique est préférentiellement logé dans le corps de lampe et est fixé à ce dernier.

L'organe élastique 29 est constitué par une ressort à spire non jointive. Le ressort prend appui sur la paroi supérieure 26. Cette paroi est percée à son centre pour le passage de la zone terminale haute de la structure support 3A. Les paliers sont montés dans les ailes d'un étrier en forme de U fixé à la paroi supérieure 26. La tringle d'actionnement 32 est logée dans l'intervale entre les réflecteurs et le corps de lampe.

Afin de pouvoir régler manuellement la concentration des rayons lumineux sur le champ opératoire, à l'arbre 31 est accouplé par l'intermédiaire d'un joint cardan une seconde tringle d'actionnement 35 accouplée par l'intermédiaire d'un joint cardan à un bouton de manoeuvre 36 externe au corps de lampe.

De façon que l'éclairage du champ opératoire soit effectué sans ombres portées, la paroi transparente 2 est constituée par plusieurs éléments radiaux dioptriques 33, identiques, disposés, côte à côte, épousant chacun le contour d'un secteur de couronne circulaire, la face supérieure 34 de chaque élément dioptrique étant une portion de surface conique de rayon de courbure décroissant depuis la bordure externe périmétrique de la paroi 2 vers son centre.

Préférentiellement, la face supérieure de chaque élément dioptrique est concave mais cette dernière pourra être convexe.

Ainsi, chaque élément dioptrique formera sur un plan une tache lumineuse épousant le contour d'un quadrilatère.

La lampe telle que décrite sera de plus équipée de capteurs de fin de course associés par exemple à l'arbre de sortie du moteur afin de matérialiser les positions extrêmes haute et basse de la structure support 3A des sources lumineuses.

Il pourra être adjoint de plus au moteur électrique 20 un limiteur de couple.

Egalement la lampe seelon l'invention pourra être équipée des moyens d'ajustement de l'intensité lumineuse de la lumière émise par la ou les sources lumineuses, ces dits moyens pouvant par exemple contrôler le courant d'alimentation des dites sources lumineuses.

De plus, autour des manchons optiques pourra être disposée une paroi athermique.

Il va de soi que tous aménagements et variantes du domaine des équivalences techniques pourront être apportés à la lampe d'éclairage telle que décrite sans pour autant sortir du cadre de l'invention tel que défini dans les revendications qui suivent.

## Revendications

1. Lampe d'éclairage chirurgical comportant un corps de lampe (1) creux monté en fixation sur une structure porteuse, doté d'une ouverture obturée par une paroi (2) transparente dans lequel sont montés au moins une source lumineuse (3) fixée à une structure support (3A) et au moins un réflecteur (4) en relation optique avec la source lumineuse pour réfléchir vers le champ opératoire les rayons lumineux émis par la source lumineuse et éclairer le dit champ opératoire lesquels rayons lumineux pour parvenir sur le dit champ traversent la paroi transparente (2), des organes de préhension manuelle (5) et (6) étant prévus sur la lampe de façon à pouvoir déplacer la lampe et ajuster sa position par rapport au champ opératoire et la source lumineuse (3) et le réflecteur (4) étant montés dans le corps de lampe (1) avec possibilité d'être déplacés l'un par rapport à l'autre par des moyens de réglage de la concentration des rayons lumineux sur le champ opératoire qui ajustent la position de la source lumineuse (3) et du réflecteur (4) l'un par rapport à l'autre de façon que l'intensité lumineuse de l'éclairage du dit champ soit optimale caractérisée en ce que le moyen de réglage automatique de la concentration des rayons lumineux sur le champ opératoire est constitué par :
- un moyen (7) sensible au touché manuel de l'opérateur, comportant au moins un élément destiné à être touché, manuellement par l'opérateur, le dit moyen (7) délivrant lorsque le dit élément est touché un signal électrique d'activation de l'opération de réglage de la concentration des rayons lumineux,
- un moyen (9) de conduite de l'opération de réglage de la concentration des rayons lumineux recevant du moyen (7) le signal d'activation de l'opération de réglage de la concentration des rayons lumineux qui n'est conduite par le moyen (9) qu'après disparition du signal d'activation, cette disparition résultant du relachement de l'organe sensible au touché,
- un moyen (10) de mesure de l'intensité lumineuse de la lumière réfléchie sur le champ opératoire activé par le moyen (9) de conduite et comportant un organe (11) sensible à la lumière placé en regard et à distance du champ opératoire délivrant une tension électrique dont la valeur est représentative de la valeur de l'intensité lumineuse de la lumière qu'il reçoit du champ opératoire,
- un premier élément (12) de mémoire couplé au moyen (10) de mesure, activé par le moyen (9) de conduite de l'opération de réglage et dans lequel lorsqu'il est activé est enregistrée la valeur de la mesure effectuée par le moyen de mesure,
- un second élément (13) de mémoire activé par le moyen (9) de conduite de l'opération de réglage et dans lequel est enregistrée la dernière mesure effectuée précédemment'par le moyen (10) de mesure,
- un moyen comparateur (14) activé par le moyen (9) de conduite de l'opération de réglage connecté par ses entrées aux éléments (12), (13) de mémoire et par sa sortie au moyen (9) de conduite, le dit moyen comparateur (14) lorsqu'il est activé comparant les deux valeurs enregistrées dans les deux éléments de mémoire (12), (13) et délivrant au moyen (9) de conduite un signal représentatif de la valeur de la différence algébrique entre la valeur de la mesure enregistrée dans la première mémoire et la valeur de la mesure enregistrée dans la seconde mémoire,
- un moyen moteur (15) commandé par le moyen (9) de conduite de l'opération de réglage, couplé mécanique ment à la structure support (3A) de la source lumineuse (3) et/ou au réflecteur (4) pour les déplacer l'un par rapport à l'autre et dans un sens ou dans l'autre, le sens de déplacement étant déterminé par le moyen (9) de conduite,
- le dit moyen (9) de conduite au cours d'une même opération de réglage de la concentration des rayons lumineux activant successivement:
a)- le moyen (10) de mesure de l'intensité,
b)- le premier élément (12) de mémoire,
c)- le moyen comparateur (14),
d)- le moyen moteur (15) ,
e)- les premier et second éléments de mémoires (12), (13) pour enregistrer dans le second élément (13) de mémoire la valeur contenue dans le premier élément (12) de mémoire, et le dit moyen (9) de conduite tant que le signal délivré par le moyen de comparaison marque une différence positive ou nulle, répétant les opération a,b,c et e tout en maintenant activé le moyen moteur (15) de façon à déplacer de manière continue la source de lumière (3) par rapport au réflecteur (4), ce mouvement de déplacement étant interrompu par désactivation du moyen moteur (15) dès que le signal délivré par le moyen de comparaison marque une différence négative.

2. Lampe d'éclairage chirurgical selon la revendication 1 caractérisée en ce que l'élément destiné à être touché du moyen sensible au touché est constitué par un des organes de préhension manuelle (5) ou (6).

3. Lampe d'éclairage chirurgical selon la revendication 1 caractérisé en ce que le dit organe de préhension manuelle constituant l'élément destiné à être touché est constitué par une poignée (5) cylindrique, stérilisable, fixée de manière amovible à la lampe et se développant suivant l'axe de cette dernière et sous la paroi transparente.

4. Lampe d'éclairage chirurgical selon la revendication 2 caractérisée en ce que le dit organe de préhension manuelle destiné à être touché est une anse (6) fixée au corps de lampe.

5. Lampe d'éclairage chirurgical selon la revendication 1 caractérisée en ce que l'organe (11) sensible à la lumière du moyen (10) de mesure de l'intensité lumineuse de la lumière réfléchie sur le champ opératoire est monté en fixation sur une platine (19) et ce dans l'axe de la lampe sous la paroi transparente (2).

6. Lampe d'éclairage chirurgical selon les revendications 3 et 5 caractérisée en ce que la poignée (5) stérilisable est creuse, est pourvue d'une ouverture en regard du champ opératoire et vient lors de la fixation sur la lampe entourer l'organe (11) sensible à la lumière.

7. Lampe d'éclairage chirurgical selon la revendication 1 caractérisée en ce que l'élément destiné à être touché du moyen sensible au touché est est une matière électriquement conductrice et est isolé de la masse de la dite lampe.

8. Lampe d'éclairage chirurgical selon la revendication 7 caractérisée en ce que le moyen (7) sensible au touché comprend un circuit électronique de détection de la variation d'intensité du courant électrique résultant du touché de l'élément destiné à être touché.

9. Lampe d'éclairage chirurgical selon la revendication 1 caractérisée en ce que le réflecteur (4) est fixe et que la source lumineuse (3) est déplaçable.

10. Lampe d'éclairage chirurgical selon la revendication 1 caractérisée en ce que :
- elle est pourvue de plusieurs sources lumineuses (3) montées à écartement les unes des autres, suivant l'axe de symétrie du corps de lampe, sur une structure support commune (3A) comportant dans sa zone terminale basse une embase,
- autour de chaque source lumineuse est disposé un manchon optique (23) présentant intérieurement une surface cylindrique d'axe confondu avec l'axe de symétrie du boîtier et extérieurement une surface de révolution convexe,
- et la structure support (3A) par son embase s'engage dans un logement de guidage en translation pratiqué dans la platine centrale (1A), et par sa zone terminale haute coopère avec des moyens d'actionnement en translation suivant l'axe de symétrie du corps de lampe.

11. Lampe d'éclairage selon la revendication 10 caractérisée en ce qu'elle est équipée de deux réflecteurs (4) à surfaces réfléchissantes tronconiques, coaxiaux, montés dans le corps de lampe l'un au dessus de l'autre et autour des manchons optiques (23).

12. Lampe d'éclairage chirurgical selon la revendication 11 caractérisée en ce que que l'angle au sommet de la surface conique suivant laquelle se développe la surface réfléchissante du réflecteur supérieur est beaucoup plus important que l'angle au sommet de la surface conique suivant laquelle se développe la surface réfléchissante de l'autre réflecteur.

13. Lampe d'éclairage selon la revendication 10 caractérisée en ce que lorsque l'une des sources lumineuses (3) est disposée dans le plan de symétrie de son manchon optique (23) les deux autres sources sont décalées par rapport au plan de symétrie de leur manchon optique (23) respectif.

14. Lampe d'éclairage chirurgical selon les revendications 10, 11, 12 et 13 prises dans leur ensemble caractérisée en ce que les faisceaux lumineux produits par les sources lumineuses en association avec leur manchon optique et leur réflecteur viennent ,se superposer ou se mélanger suivant un plan géométrique unique perpendiculaire à l'axe de la lampe.

15. Lampe d'éclairage chirurgical selon la revendication 1 caractérisée en ce que la paroi transparente (2) est constituée par plusieurs éléments radiaux dioptriques (33) identiques disposés côte à côte épousant chacun le contour d'un secteur de couronne circulaire, la face supérieure (34) de chaque élément dioptrique étant une portion de surface conique de rayon de courbure décroissant depuis la bordure externe périmétrique de la paroi (2) vers son centre.

16. Lampe d'éclairage chirurgical selon la revendication 15 caractérisée en ce que la face supérieure (34) de chaque élément (33) est concave.

17. Lampe d'éclairage chirurgical selon la revendication 15 caractérisée en ce que la face supérieure (34) de chaque élément (33) est convexe.

18. Lampe d'éclairage chirurgical selon la revendication 1 caractérisée en ce que suite à la désactivation du moyen moteur (15) lorsque le signal délivré par le moyen comparateur marque une différence négative, le moyen (9) de conduite, par réactivation du moyen moteur (15) pendant une durée prédéterminée, commande le déplacement en sens inverse de la source lumineuse (3) par rapport au réflecteur (4), et au terme de cette durée désactive le moyen moteur et interrompt l'opération de réglage.

19. Lampe d'éclairage selon la revendication 1 caractérisée en ce qu'en début de l'opération de réglage de la concentration, la source lumineuse (3) est déplacée par rapport au réflecteur (4) suivant un sens prédéterminé et le moyen (9) de conduite, par commande appropriée du moyen moteur (15), inverse ce sens de déplacement si le signal délivré par le moyen comparateur (14) marque une différence négative.
